# EUROPEAN PATENT SPECIFICATION

(11) **EP 3 964 260 B1**
(45) Date of publication and mention of the grant of the patent: **14.10.2026**
(21) Application number: 21192571.4
(22) Date of filing: 29.09.2017
(51) Int. Cl.: A61N 5/06

(54) **PHOTOTHERAPEUTIC SYSTEMS INCLUDING SPREADING AND COLLIMATING FEATURES AND RELATED TECHNOLOGY**
PHOTOTHERAPEUTISCHE SYSTEME MIT SPREIZ- UND KOLLIMATIONSMERKMALEN UND VERWANDTER TECHNOLOGIE
SYSTÈMES DE PHOTOTHÉRAPIE COMPRENANT DES CARACTÉRISTIQUES DE DIFFUSION ET DE COLLIMATION ET TECHNOLOGIE ASSOCIÉE

(30) Priority: 03.10.2016 US 201662403590 P
(43) Date of publication of application: 09.03.2022
(62) Document number(s) of the earlier application(s) in accordance with Art. 76 EPC: 17858943.8 / 3 519 048
(73) Proprietor: Solius Labs, Inc., Bainbridge Island, WA 98110 (US)
(72) Inventor: MOFFAT, Willian Alexander, Bainbridge Island, WA98110 (US); KIRKWOOD, Keith, Bainbridge Island, WA98110 (US); STEWART, Jeff, Bainbridge Island, WA98110 (US); ALLISON, Jeff, Bainbridge Island, WA98110 (US); SHAY, Brian, Bainbridge Island, WA98110 (US); NESTOROVIC, Ned, Bainbridge Island, WA98110 (US)
(74) Representative: Forresters IP LLP

(56) References cited:
- WO-A1-2016/127120
- US-A1- 2010 331 929
- US-A1- 2011 299 056
- US-A1- 2013 172 963
- US-A1- 2015 217 132
- US-B2- 7 638 780

## Description

### TECHNICAL FIELD

The present technology is related to phototherapeutic systems well-suited for vitamin D phototherapy and treating skin conditions.

### BACKGROUND

Vitamin D refers to a group of fat-soluble secosteroids that the human body can synthesize through adequate exposure to sunlight or UV radiation. More specifically, previtamin D3 is made in the skin when 7-dehydrocholesterol ("7-DHC") reacts with ultraviolet B ("UVB") light. Vitamin D can also be absorbed from the various dietary sources, such as fatty fish (e.g., salmon and tuna), vitamin D fortified foods (e.g., dairy and juice products), and vitamin D supplements. Once absorbed, vitamin D travels through the bloodstream to the liver where it is converted into the prohormone calcidiol. The calcidiol is, in turn, converted into calcitriol (the hormonally active form of vitamin D) by the kidneys or monocyte-macrophages in the immune system. When synthesized by the monocyte-macrophages, calcitriol acts locally as a cytokine to defend the body against microbial invaders. Kidney-synthesized calcitriol circulates through the body to regulate the concentration of calcium and phosphate in the bloodstream, and thereby promotes adequate mineralization, growth, and reconstruction of the bones. Therefore, an inadequate level of vitamin D, (typically characterized by a calcidiol concentration in the blood of less than 20-40 ng/m²) can cause various bone softening diseases, such as rickets in children and osteomalacia in adults. Vitamin D deficiency has also been linked to numerous other diseases and disorders, such as depression, heart disease, gout, autoimmune disorders, and a variety of different cancers.

Physicians have recommended vitamin D supplements as a preventative measure to increase vitamin D levels. The American Institute of Medicine, for example, recommends a daily dietary vitamin D intake of 600 international units (IU) for those 1-70 years of age, and 800 IU for those 71 years of age and older. Other institutions have recommended both higher and lower daily vitamin D doses. The limitations on daily dosages also reflect an effort to prevent ingesting too much vitamin D, which can eventually become toxic. In contrast, the human physiology has adapted to significantly higher daily doses of vitamin D from sunlight (e.g., 4,000-20,000 IU/day or more). UVB radiation has been identified as a more desirable source of vitamin D because of the ease at which vitamin D is produced from exposure to sunlight and the body's natural ability to inhibit excessive vitamin D intake through the skin.

The International Commission on Illumination (also known as Le Commission Internationale de l'Eclairage ("CIE")) has created two standardized action spectrums associated with UV radiation and vitamin D production: "The Erythema Reference Action Spectrum and Standard Erythema Dose" (ISO 7166:1999), used to determine erythema (i.e., sunburn) response to individual wavelengths from 250 nm to 400 nm; and "The Action Spectrum for the Production of Previtamin D3 in Human Skin" (CIE 174:2006), used to determine the conversion efficiency of 7-DHC to previtamin D3 at individual wavelengths from 255 nm to 320 nm. After 7-DHC is converted to previtamin D3, it may be photoisomerized to either of two inert products, lumisterol or tachysterol, or it can undergo a reverse reaction and revert back to 7-DHC. These photoreactions are driven by continued UV radiation, but the absorption spectra of each photoproduct varies. A study used to create the CIE previtamin D3 action spectrum standardized the UV dosage to limit the conversion of 7-DHC to previtamin D3 to less than 5% to help mitigate any photoisomerization of previtamin D3 to photoproducts (e.g., lumisterol, tachysterol, and 7-DHC).

### BRIEF DESCRIPTION OF THE DRAWINGS

Many aspects of the present technology can be better understood with reference to the following drawings. The components in the drawings are not necessarily to scale. Instead, emphasis is placed on illustrating clearly the principles of the present technology. For ease of reference, throughout this disclosure identical reference numbers may be used to identify identical, similar, or analogous components or features of more than one embodiment of the present technology.
Figure 1 is a partially schematic side view of a phototherapeutic system in accordance with an embodiment of the present technology.
Figure 2 is an enlarged partially schematic side view of a portion of the phototherapeutic system shown in Figure 1.
Figure 3 is an enlarged partially schematic side view of a portion of a phototherapeutic system in accordance another embodiment of the present technology.
Figure 4 is a partially schematic top plan view of a phototherapeutic system in accordance with another embodiment of the present technology.
Figures 5A and 5B are partially schematic side views of radiation sources that can be used in phototherapeutic systems in accordance additional embodiments of the present technology.
Figure 5C schematically illustrates a phototherapeutic system including any of the radiation sources shown in Figures 5A and 5B in accordance with another embodiment of the present technology.
Figures 6A and 6B are partially schematic side views of radiation sources that can be used in wearable phototherapeutic devices in accordance additional embodiments of the present technology.
Figure 6C schematically illustrates a wearable phototherapeutic device including any of the radiation sources shown in Figures 6A and 6B in accordance with another embodiment of the present technology.
Figure 7 is a flow chart illustrating a phototherapeutic method in accordance with an embodiment of the present technology.

### DETAILED DESCRIPTION

The invention relates to a radiation source as defined in the appended claims. Embodiments, examples or aspects in the following disclosure, in particular methods, which do not fall under the scope of the claims are presented for illustration purposes only and do not form part of the invention.

Phototherapeutic systems, devices, and methods in accordance with at least some embodiments of the present technology include innovative features for spreading UV radiation and delivering the UV radiation to a human subject with a high degree of uniformity. A phototherapeutic system in accordance with a particular embodiment includes a UV radiation source configured to direct at least partially collimated UV radiation along a radiation path that extends to an irradiation zone configured to accommodate a human subject. The system further comprises a filter, a spreader, and a collimator disposed successively farther downstream from the radiation source along the radiation path. The spreader and the collimator respectively decrease and increase collimation of the UV radiation before the UV radiation reaches the subject. In at least some cases, UV radiation from a relatively small number of high-intensity sources is efficiently filtered and delivered to a subject with uniformity at least approaching that of natural sunlight. Furthermore, this high uniformity can be independent of the subject's position within the irradiation zone. Thus, phototherapeutic systems in accordance with at least some embodiments of the present technology are more practical, more effective, and/or offer a better user experience relative to conventional counterparts. Phototherapeutic systems in accordance with embodiments of the present technology can have these and/or other advantages relative to conventional counterparts whether or not such advantages are described herein.

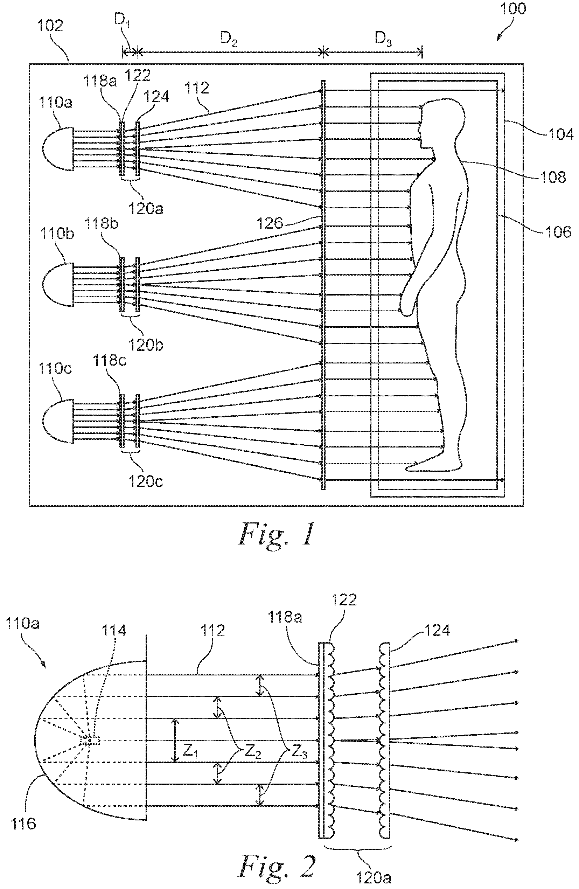

Specific details of phototherapeutic systems, devices, and methods in accordance with several embodiments of the present technology are described herein with reference to Figures 1-7. Although phototherapeutic systems, devices, and methods may be described herein primarily or entirely in the context of vitamin D phototherapy, other contexts in addition to those described herein are within the scope of the present technology. For example, suitable features of described phototherapeutic systems can be implemented in the context of tanning, treatment of skin conditions (e.g., psoriasis), treatment of autoimmune conditions (e.g., Crohn's disease, rheumatoid arthritis, etc.), and/or treatment of seasonal affective disorder, among other examples. Furthermore, it should be understood, in general, that other systems, devices, and methods in addition to those disclosed herein are within the scope of the present technology. For example, systems, devices, and methods in accordance with embodiments of the present technology can have different and/or additional configurations, components, procedures, etc. than those disclosed herein. Moreover, a person of ordinary skill in the art will understand that systems, devices, and methods in accordance with embodiments of the present technology can be without various configurations, components, procedures, etc. disclosed herein without deviating from the present technology.

Figure 1 is a partially schematic side view of a phototherapeutic system 100 in accordance with an embodiment of the present technology, and Figure 2 is an enlarged partially schematic side view of a portion of the phototherapeutic system 100. With reference to Figure 1, the phototherapeutic system 100 can include a housing 102 at least partially defining a walk-in booth 104 (e.g., kiosk) and an irradiation zone 106 within the booth 104. In the illustrated embodiment, the booth 104 and the irradiation zone 106 are configured to fully receive a human subject 108 and to contain radiation within the booth 104. In other embodiments, counterparts of the booth 104 and the irradiation zone 106 can be configured to receive only a portion of the subject 108. For example, counterparts of the booth 104 and the irradiation zone 106 can be configured to receive only a torso, arm, leg, etc. of the subject 108. Furthermore, counterparts of the booth 104 and the irradiation zone 106 can be configured to receive the subject 108 in a supine, prone, reclined, or other suitable position rather than in the illustrated standing position.

As shown in Figure 1, the phototherapeutic system 100 can include UV radiation sources 110 (individually identified as radiation sources 110a-110c) configured to direct UV radiation along corresponding radiation paths 112 (schematically represented by lines) extending from the radiation sources 110 to the irradiation zone 106. The radiation source 110a will now be described in additional detail with the understanding that the radiation sources 110b, 110c may have the same or similar features. As shown in Figure 2, the radiation source 110a can be configured to direct at least partially collimated UV radiation along the corresponding radiation path 112. For example, the radiation source 110a can include a concentrated source 114 and a collimating reflector 116 shaped, positioned, or otherwise configured to reflect UV radiation generated at the concentrated source 114 and thereby increase collimation of this UV radiation. The concentrated source 114 can be a volume of plasma maintained by an applied voltage. For example, the concentrated source 114 can be a mercury-vapor lamp, a metal-halide lamp, a xenon short-arc lamp, or another suitable type of high-intensity discharge lamp. Alternatively, the concentrated source 114 can generate the UV radiation in another suitable manner. For example, counterpart light-emitting diode concentrated sources are described below in the context of another embodiment of the present technology.

With reference to Figures 1 and 2 together, the phototherapeutic system 100 can further include filters 118 (individually identified as filters 118a-118c) disposed along the radiation paths 112 downstream from the radiation sources 110. The filters 118 can be configured to preferentially permit passage of UV radiation with relatively high potential to promote vitamin D production and/or relatively low potential to cause erythema. Correspondingly, the filters 118 can be configured to preferentially block passage of UV radiation with relatively low potential to promote vitamin D production and/or relatively high potential to cause erythema. For example, the filters 118 can be configured to preferentially permit passage of UV radiation at a wavelength of about 297 nm (e.g., 292 nm to 304 nm, and in particular 297 nm +/- 3 nm). Other suitable features and alternative forms of the housing 102, the booth 104, the irradiation zone 106, the radiation sources 110, and the filters 118 of the phototherapeutic system 100 are described in the applications PCT/ US2013/020179, PCT/US2014/062352, PCT/US2016/016873, and PCT/US2016/029615.

In at least some cases, it is advantageous for the filters 118 to receive collimated UV radiation rather than non-collimated UV radiation. For example, transmission of UV radiation through the filters 118 may depend on both wavelength and angle of incidence. If the filters 118 receive UV radiation at a wide range of non-normal angles of incidence it may be difficult to filter consistently all but a narrow range of wavelengths. Conversely, if the filters 118 receive UV radiation predominantly at a normal angle of incidence, it can be relatively straightforward to achieve this functionality. Although advantageous for filtering, collimation of UV radiation may be disadvantageous for efficient delivery of the UV radiation to a large area. For example, the filtered, collimated UV radiation emanating from the radiation sources 110 and the filters 118, without further processing, would be concentrated in only a small zone Z₁ of the beam because the center of the beam of collimated light would have a much higher intensity than the outer portion of the beam. Outer zones Z₂ and Z₃ of the beam generally have decreasing intensity. As a result, the dose of radiation delivered to the subject 108 in the outer zones Z₂ and Z₃ of the beam would be limited by the amount of radiation that produces an unacceptable level of exposure in the small area of the subject illuminated by the central zone Z₁ of the beam.

To promote efficient delivery of filtered UV radiation to a large area and/or for other reasons, the phototherapeutic system 100 can include spreaders 120 (individually identified as spreaders 120a-120c) disposed along the radiation paths 112 downstream from the filters 118. In some embodiments, the spreaders 120a-120c carry the corresponding filters 118a-118c. For example, the filters 118a-118c can include films overlying flat upstream surfaces of the corresponding spreaders 120a-120c. In other embodiments, counterparts of the filters 118 can be supported independently from the spreaders 120, such as being carried by corresponding counterparts of the radiation sources 110a-110c, or the filters 118 can have other suitable configurations. The filters 118 can be oriented to receive UV radiation at a zero degree angle of incidence. Accordingly, the filters 118 can be carried by flat surfaces oriented to be perpendicular to immediately upstream portions of the radiation paths 112, such as flat upstream surfaces of the spreaders 120 having this orientation.

As shown in Figure 1, the spreaders 120 can expand the filtered, collimated UV radiation downstream from the filters 118 so that the UV radiation extends throughout at least one side of the irradiation zone 106. In at least some cases, the spreaders 120 expand the UV radiation without scattering the UV radiation to any significant degree. The spreaders 120a-120c can include a first array of lenslets 122 and a second array of lenslets 124 spaced apart from one another along the radiation paths 112 by a distance D₁. The first array of lenslets 122 can refract the UV radiation to form a beam having a first level of divergence. The second array of lenslets 124 can further refract the UV radiation to form a beam having a second level of divergence greater than the first level of divergence. The distance D₁ can be adjusted to change the overall degree of divergence of the UV radiation within the phototherapeutic system 100, such as to change the size of the irradiation zone 106. As shown in Figure 2, in the illustrated embodiment, the first array of lenslets 122 has a planar upstream surface and a convex downstream surface, and the second array of lenslets 124 has a convex upstream surface and a planar downstream surface. In other embodiments, the first and second arrays of lenslets 122, 124 can have other suitable configurations. Furthermore, one or both of the first and second arrays of lenslets 122, 124 can carry optical films (e.g., coatings) other than the filters 118. For example, one or both of the first and second arrays of lenslets 122, 124 can carry antireflective films and/or films that modify optical density.

With reference to Figure 1, at a certain distance (D₂) along the radiation paths 112 downstream from the spreaders 120, the UV radiation can achieve a desirable (e.g., optimal) level of spreading to provide relative uniformity across the skin surface. Unfortunately, in the context of phototherapy, D₂ is unlikely to correspond to the actual distance between the spreaders 120 and the subject 108 throughout the irradiation zone 106. For example, the subject's body shape and position within the irradiation zone 106 may be difficult to predict and may vary considerably from one subject to another. Furthermore, the subject 108 may move during a phototherapy session. These and other factors can affect the distance between the spreaders 120 and a given portion of the subject's skin. Even if the spreaders 120 are adjusted to cause D₂ to be within an expected range of distances between the spreaders 120 and the subject 108, the actual distance between the spreaders 120 and some portions of the subject 108 will be less than D₂, and the actual distance between the spreaders 120 and other portions of the subject 108 will be greater than D₂. Portions of the subject 108 closer to the spreaders 120 may be between diverging beams of UV radiation, thus receiving less than a desirable level of exposure or may be within a diverging beam of UV radiation, thus receiving more than a desirable level of exposure. Other portions of the subject 108 farther from the spreaders 120 may be at overlapping portions of diverging beams of UV radiation, thus also receiving more than a desirable level of exposure.

To promote uniform delivery of filtered UV radiation to the subject 108 and/or for other reasons, the embodiment of the phototherapeutic system 100 shown in Figure 1 further includes a collimator 126 disposed along the radiation paths 112 downstream from the spreaders 120. In at least some embodiments, the collimator 126 includes a Fresnel lens. The collimator 126 increases collimation of UV radiation downstream from the spreaders 120 such that further divergence of the UV radiation between the collimator 126 and the subject 108 is reduced or eliminated. Thus, over the length of the radiation paths 112, the UV radiation originating at the concentrated source 114 is collimated at the collimating reflectors 116, spread at the spreaders 120, and re-collimated at the collimator 126. By collimating the UV radiation after the spreaders 120, the UV radiation can be delivered to the subject 108 uniformly irrespective of the distance (D₃) between the collimator 126 and the subject 108 within the irradiation zone 106.

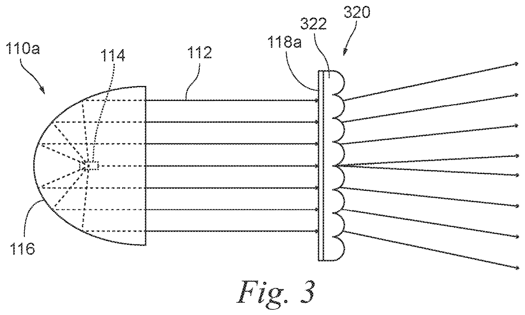

Figure 3 illustrates an alternative embodiment of a spreader 320 including a single lenslet array 322 instead of the spreaders 120 (Figure 1) that have a plurality of lenslet arrays 122 and 124. The lenslet array 322 is thicker than the lenslet arrays 122 and 124 individually, and thus it may be possible to achieve the same level of divergence of UV radiation from a given source using the single lenslet array 322 of relatively thick material rather than using multiple, spaced-apart lenslet arrays of relatively thinner material. In systems in accordance with still other embodiments of the present technology, counterparts of the radiation sources 110, the spreaders 120, and other components of the phototherapeutic system 100 can have other suitable forms.

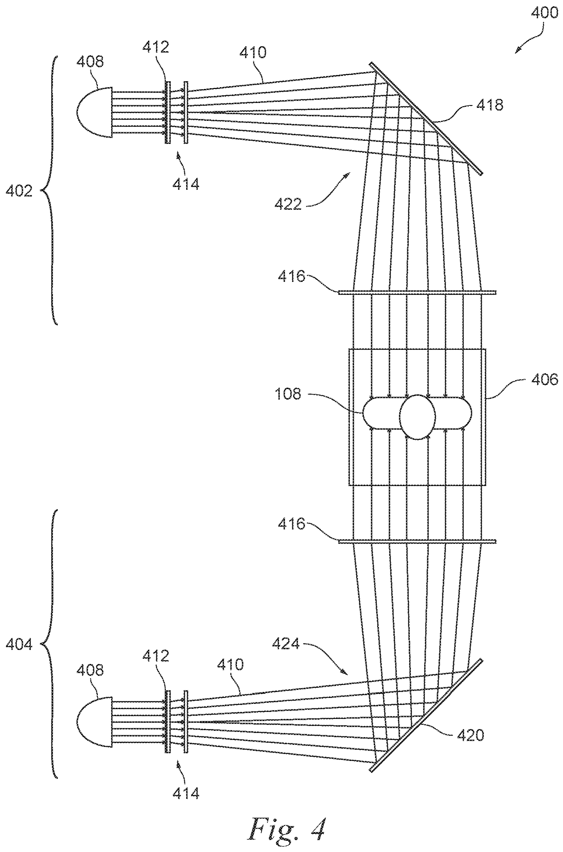

Figure 4 is a partially schematic top plan view of a phototherapeutic system 400 in accordance with another embodiment of the present technology. The phototherapeutic system 400 can include a first UV radiation assembly 402 and a second UV radiation assembly 404 configured to provide UV radiation to an irradiation zone 406 via opposite respective sides of the irradiation zone 406. Individually, the first and second UV radiation assemblies 402, 404 can include a radiation source 408, radiation paths 410, a filter 412, a spreader 414, and a collimator 416 similar to the corresponding features of the phototherapeutic system 100 (Figure 1). In addition to these components, the first and second UV radiation assemblies 402, 404 can respectively include first and second reflectors 418, 420. The first and second reflectors 418, 420 can be configured to create first and second folds 422, 424 in the corresponding radiation paths 410 downstream from the corresponding spreaders 414 and upstream from the corresponding collimators 416. The first and second reflectors 418, 420 can be advantageous, for example, to increase the effective distance between the corresponding spreaders 414 and collimators 416 in a smaller footprint compared to a counterpart system having a straight radiation path without the first and second folds 422, 424. This can allow for a greater degree of divergence of UV radiation approaching the irradiation zone 406 without requiring that the overall phototherapeutic system 400 be unduly long.

In the illustrated embodiment, the first and second reflectors 418, 420 fold the corresponding radiation paths 410 at right angles. In other embodiments, the first and second reflectors 418, 420 can fold the corresponding radiation paths 410 at other suitable angles. Furthermore, additional reflectors can be incorporated along the radiation paths 410 to further extend the distances between the spreaders 414 and collimators 416 within a desired footprint for the phototherapeutic system 400. Phototherapeutic systems in accordance with still other embodiments can include only one of the first and second UV radiation assemblies 402, 404 or more than two UV radiation assemblies with or without folding reflectors. Individually, the first and second UV radiation assemblies 402, 404 can include any suitable number of radiation sources 408. Features such as the angles of the reflectors, the number of reflectors, the orientations of the UV radiation assemblies, and the number of UV radiation assemblies, among other features, can be modified to achieve desirable form factors in phototherapeutic systems in accordance with various embodiments of the present technology.

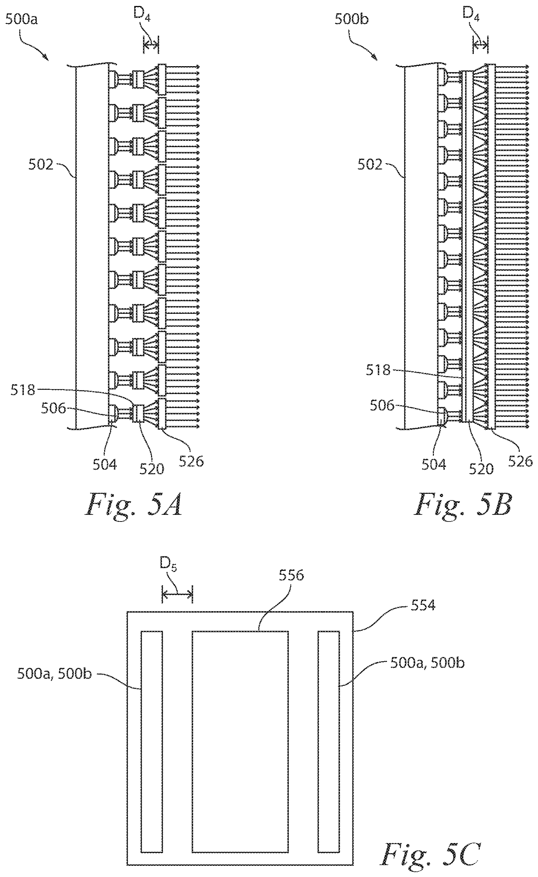

Figures 5A and 5B are partially schematic side views of radiation sources 500a and 500b, respectively, that can be used in phototherapeutic systems in accordance additional embodiments of the present technology. The radiation sources 500a, 500b include a light-emitting diode (LED) array 502 instead of the radiation sources 110 (Figure 1). The LED array 502 can include a plurality of LEDs 504 that act as concentrated sources for generating UV radiation. The radiation sources 500a, 500b can further include collimating lenses 506 attached to the LEDs 504 or spaced immediately downstream from the LEDs 504. The collimating lenses 506 can be configured so that each LED 504 has a corresponding collimating lens 506. The collimating lenses 506 are configured to refract UV radiation generated at the LEDs 504 and thereby increase collimation of the UV radiation, and thus they can serve a purpose similar to that of the collimating reflector 116 (Figure 2).

The radiation sources 500a, 500b can further include one or more filters 518 and spreaders 520 aligned with the individual LEDs 504. Although the LEDs 504 can emit radiation in a narrow bandwidth (e.g., 15-20 nm), several phototherapeutic procedures benefit from even narrower bandwidths (e.g., 2-5 nm). As such, the filters 518 are still generally desirable for use with the LEDs 504 in the radiation sources 500a, 500b. In the embodiment of the radiation source 500a, individual filters 518 and spreaders 520 are aligned with a corresponding LED 504. In the embodiment of the radiation source 500b, a single array-sized filter 518 and spreader 520 are used. The radiation sources 500a, 500b also include at least one collimator 526 spaced apart from the spreaders 520 by a distance D₄. For example, the radiation source 500a has a plurality of collimators 526 with each collimator 526 being aligned with a corresponding LED 504, while the radiation source 500b has a single array-sized collimator 526. The distance D₄ is set such that radiation from one LED 504 begins to overlap radiation from adjacent LEDs 504. As a result, the radiation from the LEDs 504 is spread to the point it is at least substantially uniform, and then the radiation is re-collimated at the collimator 526.

Because the LEDs 504 are generally small, a large number of LEDs 504 can be spaced relatively close to each other so that the LED array 502 is a large panel of concentrated sources that each produce a common distribution of radiation. The LED array 502 thus produces a relatively more uniform distribution of light at a close distance to the LED array 502 compared to the radiation sources 110 (Figure 1). As a result, embodiments using an LED array 502 as the radiation source do not necessarily need to space the spreaders 520 as far apart from the collimators 526 as compared to the radiation sources 110 that have a highly nonuniform distribution of radiation.

Figure 5C schematically illustrates a phototherapeutic system 550 having a booth 554, a radiation zone 556, and any of the radiation sources 500a and/or 500b. In this embodiment, the radiation sources 500a, 500b are spaced apart from the radiation zone 556 by a distance D₅ that is much shorter than the distance D₁+D₂+D₃ between the radiation sources 110a-110c and the radiation zone 106 illustrated in Figure 1. This is expected to reduce the size of the booth 554 compared to the booth 104 illustrated in Figure 1 or the booth necessary for the phototherapeutic system 400 illustrated in Figure 4. This can be a significant advantage because floor space is often a limiting factor in many types of installations. In other embodiments, the booth 104 can be replaced with a wearable substrate (e.g., a blanket, a garment, a cuff, etc.) configured for close contact with a subject's skin.

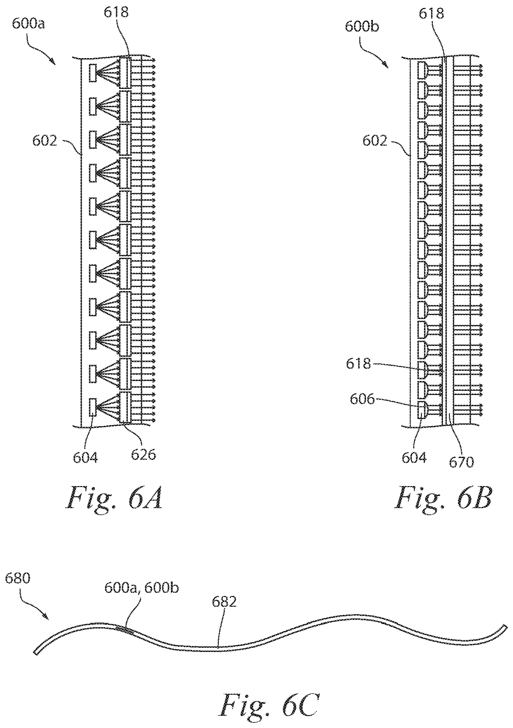

Figures 6A and 6B are partially schematic side views of radiation sources 600a and 600b, respectively, that can be used in phototherapeutic systems in accordance additional embodiments of the present technology. Similar to the radiation sources 500a, 500b (Figures 5A and 5B), the radiation sources 600a, 600b include a light-emitting diode (LED) array 602 including a plurality of LEDs 604 that act as concentrated sources for generating UV radiation. The radiation source 600a includes collimators 626 downstream from the LEDs 604, and a plurality of filters 618 downstream from the collimators 626. The radiation source 600b includes collimating lenses 606 attached to the LEDs 604 or spaced immediately downstream from the LEDs 604, and an array-sized filter 618 downstream from the collimating lenses 606. The radiation source 600b further includes a transparent backing 670 carrying the filter 618. Figure 6C schematically illustrates a wearable phototherapeutic device 680 having a flexible substrate 682 and any of the radiation sources 600a and/or 600b carried by (e.g., embedded in) the flexible substrate 682. As with wearable counterparts of the booth 554 (Figure 5C), the phototherapeutic device 680 can be a blanket, garment, cuff, or have another suitable form configured for close contact with a subject's skin.

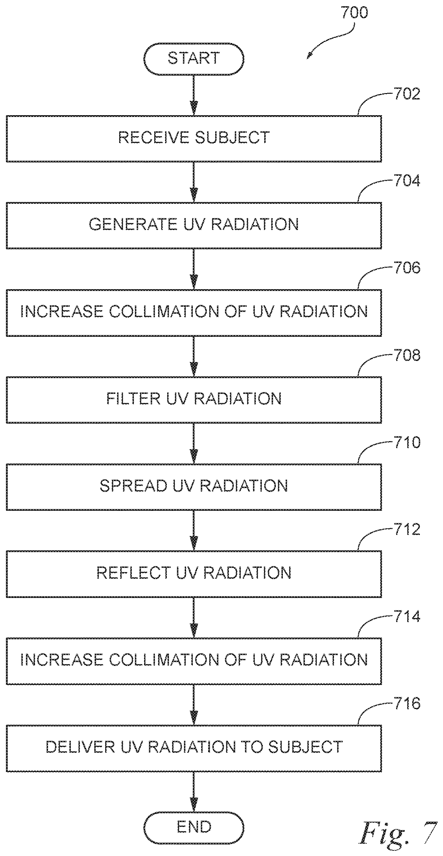

Figure 7 is a flow chart illustrating a phototherapeutic method 700 in accordance with an embodiment of the present technology. With reference to Figures 1-7 together, the method 700 can include receiving the subject 108 at the booth 104 (block 702). The method 700 can further include directing at least partially collimated UV radiation along the radiation paths 712, 710 to a skin surface of the subject 108 while the subject 108 is within the booth 104. In at least some cases, this includes generating the UV radiation at the concentrated sources 114 (block 704) and increasing collimation of the generated UV radiation (block 706). Increasing collimation of the generated UV radiation can occur by reflecting the UV radiation at the collimating reflectors 116, by refracting the UV radiation at the collimating lenses 506, or in another suitable manner. Next, the method 700 can include filtering the UV radiation directed along the radiation paths 112, 410 (block 708). When the method 700 is applied in the context of vitamin D phototherapy and in other cases, filtering the UV radiation can include preferentially permitting passage of UV radiation at a wavelength of about 297 nm relative to UV radiation at wavelengths greater than 304 nm and/or less than 292 nm.

After filtering the UV radiation, the method 700 can include spreading (e.g., spreading without scattering) the filtered UV radiation (block 710) and thereby decreasing collimation of the filtered UV radiation. Spreading the filtered UV radiation can occur at the first and second arrays of lenslets 122, 124. After passing through the first and second arrays of lenslets 122, 124, the UV radiation can form diverging beams. The method 700 can include reflecting these beams toward the skin surface of the subject 108 (block 712) and thereby folding the beams. Before the beams reach the skin surface of the subject 108, the method 700 can include increasing collimation of the UV radiation at the collimator 126, 416 (block 714). The UV radiation can then be delivered to the subject 108 (block 716) in a desirable manner, such as with high uniformity independent of the subject's position within the booth 104.

### Conclusion

This disclosure is not intended to be exhaustive or to limit the present technology to the precise forms disclosed herein. Although specific embodiments are disclosed herein for illustrative purposes, various equivalent modifications are possible without deviating from the present technology as set forth in the claims, as those of ordinary skill in the relevant art will recognize. In some cases, well-known structures and functions have not been shown and/or described in detail to avoid unnecessarily obscuring the description of the embodiments of the present technology as set forth in the claims.

Although steps of methods may be presented herein in a particular order, in alternative embodiments the steps may have another suitable order. Similarly, certain aspects of the present technology disclosed in the context of particular embodiments can be combined or eliminated in other embodiments. Furthermore, while advantages associated with certain embodiments may have been disclosed in the context of those embodiments, other embodiments may also exhibit such advantages, and not all embodiments need necessarily exhibit such advantages or other advantages disclosed herein to fall within the scope of the present technology as set forth in the claims.

Throughout this disclosure, the singular terms "a," "an," and "the" include plural referents unless the context clearly indicates otherwise. Similarly, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Additionally, the terms "comprising" and the like may be used herein to mean including at least the recited feature(s) such that any greater number of the same feature(s) and/or one or more additional types of features are not precluded. Directional terms, such as "upper," "lower," "front," "back," "vertical," and "horizontal," may be used herein to express and clarify the relationship between various elements. It should be understood that such terms do not denote absolute orientation. Reference herein to "one embodiment," "an embodiment," or similar formulations means that a particular feature, structure, operation, or characteristic described in connection with the embodiment can be included in at least one embodiment of the present technology. Thus, the appearances of such phrases or formulations herein are not necessarily all referring to the same embodiment. Furthermore, various particular features, structures, operations, or characteristics may be combined in any suitable manner in one or more embodiments of the present technology.

## Claims

1. A radiation source (500a) for use with a phototherapeutic system (550), the radiation source (500a) comprising:
a plurality of light-emitting diodes (LEDs) (504) carried by a housing, each of the plurality of LEDs (504) configured to direct UV radiation along a respective radiation path extending from the associated LED (504) to an irradiation zone (556), each of the plurality of LEDS (504) configured to emit UV radiation to stimulate vitamin D production in skin of a human subject;
a plurality of spreaders (520), wherein one spreader (520) of the plurality of spreaders (520) is disposed along each of the radiation paths downstream from the respective LEDs (504); and
a plurality of collimators (526), wherein one collimator (526) of the plurality of collimators (526) is disposed along each of the radiation paths downstream from the respective spreaders (520).

2. The radiation source (500a) of claim 1 wherein the plurality of LEDs (504) form a panel of concentrated sources that each produce a common distribution of the UV radiation.

3. The radiation source (500a) of any one of the preceding claims wherein the plurality of LEDs (504) are arranged in an array.

4. The radiation source (500a) of any one of the preceding claims wherein the plurality of LEDs (504) are spaced apart from each other such that radiation from one LED (504) overlaps radiation emitted from an adjacent LED (504).

5. The radiation source (500a) of any one of the preceding claims wherein the housing is configured to carry the plurality of LEDs (504) in a vertical arrangement, the irradiation zone (556) configured to accommodate at least a body part of the human subject, wherein the body part is in a corresponding vertical arrangement.

6. The radiation source (500a) of any one of the preceding claims wherein the housing defines the irradiation zone, wherein the irradiation zone is configured to accommodate a portion of the human subject.

7. The radiation source (500a) of any one of the preceding claims wherein:
the plurality of LEDs (504) are configured to direct at least partially collimated UV radiation along the radiation path;
the plurality of spreaders (520) decrease collimation of UV radiation from the plurality of LEDs (504); and
the plurality of collimators (526) increase collimation of UV radiation from the plurality of LEDs (504).

8. The radiation source (500a) of any one of the preceding claims wherein each of the radiation paths includes a reflector positioned downstream of the respective LED (504), the reflector configured to direct the UV radiation along the respective radiation path.

9. The radiation source (500a) of any one of the preceding claims, further comprising a filter (518) disposed along each of the radiation paths that is configured to preferentially permit passage of UV radiation at a wavelength of about 297 nm relative to UV radiation at wavelengths greater than 304 nm or less than 292 nm.

10. The radiation source (500a) of claim 9 wherein the filter (518) includes a film.

11. The radiation source (500a) of any one of the preceding claims, further comprising a plurality of reflectors configured to reflect UV radiation generated at the plurality of LEDs, the reflected radiation directed along the respective radiation path extending from the respective LED toward the irradiation zone.

12. The radiation source (500a) of claim 1-10, further comprising a filter disposed along each of the radiation paths that is positioned downstream of the respective LED.

13. The radiation source (500a) of any of the claims 1-11, further comprising a filter disposed along each of the radiation paths that is positioned downstream of the respective collimator.

14. The radiation source (500a) of any one of the preceding claims, further comprising a plurality of lenses (506) attached to respective emitting sides of the plurality of LEDs (504).

15. The radiation source (500a) of any one of the preceding claims, further comprising a lens positioned downstream of the each of the plurality of LEDs (504).

## Patentansprüche

1. Strahlungsquelle (500a) zur Verwendung mit einem fototherapeutischen System (550), wobei die Strahlungsquelle (500a) Folgendes umfasst:
Eine Vielzahl von Leuchtdioden (LEDs) (504), die von einem Gehäuse getragen werden, wobei jede der Vielzahl von LEDs (504) dazu konfiguriert ist, UV-Strahlung entlang eines jeweiligen Strahlengangs zu lenken, der von der zugehörigen LED (504) zu einer Bestrahlungszone (556) verläuft, wobei jede der Vielzahl von LEDs (504) dazu konfiguriert ist, UV-Strahlung zu emittieren, um die Vitamin-D-Produktion in der Haut eines menschlichen Subjekts zu stimulieren;
eine Vielzahl von Streuelementen (520), wobei ein Streuelement (520) der Vielzahl von Streuelementen (520) entlang jedes der Strahlengänge stromabwärts der jeweiligen LEDs (504) angeordnet ist; und
eine Vielzahl von Kollimatoren (526), wobei ein Kollimator (526) der Vielzahl von Kollimatoren (526) entlang jedes der Strahlengänge stromabwärts der jeweiligen Streuelemente (520) angeordnet ist.

2. Strahlungsquelle (500a) nach Anspruch 1, wobei die mehreren LEDs (504) ein Feld konzentrierter Quellen bilden, die jeweils eine gemeinsame Verteilung der UV-Strahlung erzeugen.

3. Strahlungsquelle (500a) nach einem der vorhergehenden Ansprüche, wobei die Vielzahl von LEDs (504) in einem Array angeordnet sind.

4. Strahlungsquelle (500a) nach einem der vorhergehenden Ansprüche, wobei die Vielzahl von LEDs (504) derart voneinander beabstandet sind, dass sich die Strahlung einer LED (504) mit der von einer benachbarten LED (504) emittierten Strahlung überlappt.

5. Strahlungsquelle (500a) nach einem der vorhergehenden Ansprüche, wobei das Gehäuse dazu konfiguriert ist, die Vielzahl von LEDs (504) in einer vertikalen Anordnung zu tragen, wobei die Bestrahlungszone (556) dazu konfiguriert ist, zumindest einen Körperteil der menschlichen Person aufzunehmen, wobei sich der Körperteil in einer entsprechenden vertikalen Anordnung befindet.

6. Strahlungsquelle (500a) nach einem der vorhergehenden Ansprüche, wobei das Gehäuse die Bestrahlungszone definiert, wobei die Bestrahlungszone dazu konfiguriert ist, einen Teil des menschlichen Subjekts aufzunehmen.

7. Strahlungsquelle (500a) nach einem der vorhergehenden Ansprüche, wobei:
Die Vielzahl von LEDs (504) ist dazu konfiguriert, zumindest teilweise kollimierte UV-Strahlung entlang des Strahlengangs zu lenken;
die Vielzahl von Streuelementen (520) die Kollimation der UV-Strahlung der Vielzahl von LEDs (504) verringert; und
die Vielzahl von Streuelementen (526) die Kollimation der UV-Strahlung der Vielzahl von LEDs (504) erhöht; und

8. Strahlungsquelle (500a) nach einem der vorhergehenden Ansprüche, wobei jeder der Strahlengänge einen Reflektor umfasst, der stromabwärts der jeweiligen LED (504) positioniert ist, wobei der Reflektor dazu konfiguriert ist, die UV-Strahlung entlang des jeweiligen Strahlengangs zu lenken.

9. Strahlungsquelle (500a) nach einem der vorhergehenden Ansprüche, ferner einen entlang jedes der Strahlengänge angeordneten Filter (518) umfassend, der dazu konfiguriert ist, den Durchgang von UV-Strahlung mit einer Wellenlänge von etwa 297 nm gegenüber UV-Strahlung mit Wellenlängen von mehr als 304 nm oder weniger als 292 nm bevorzugt zu ermöglichen.

10. Strahlungsquelle (500a) nach Anspruch 9, wobei der Filter (518) einen Film umfasst.

11. Strahlungsquelle (500a) nach einem der vorhergehenden Ansprüche ferner eine Vielzahl von Reflektoren umfassend, die dazu konfiguriert sind, von der Vielzahl von LEDs erzeugte UV-Strahlung zu reflektieren, wobei die reflektierte Strahlung entlang des jeweiligen, von der jeweiligen LED zur Bestrahlungszone verlaufenden Strahlengangs gelenkt wird.

12. Strahlungsquelle (500a) nach einem der Ansprüche 1 bis 10, ferner einen entlang jedes der Strahlengänge angeordneten, Filter umfassend, der stromabwärts der jeweiligen LED positioniert ist.

13. Strahlungsquelle (500a) nach einem der Ansprüche 1-11 ferner einen Filter umfassend, der entlang jedes der Strahlengänge angeordnet ist, der stromabwärts des jeweiligen Kollimators positioniert ist.

14. Strahlungsquelle (500a) nach einem der vorhergehenden Ansprüche ferner eine Vielzahl von Linsen (506) umfassend, die an den jeweiligen Emissionsseiten der Vielzahl von LEDs (504) angebracht sind.

15. Strahlungsquelle (500a) nach einem der vorhergehenden Ansprüche ferner eine Linse umfassend, die stromabwärts von jeder der Vielzahl von LEDs (504) angeordnet ist.

## Revendications

1. Source de rayonnement (500a) destinée à être utilisée avec un système de photothérapie (550), la source de rayonnement (500a) comprenant :
une pluralité de diodes électroluminescentes (LED) intégrée à un boîtier, chaque LED de la pluralité de LED (504) étant configurée pour diriger des rayons UV le long d'un trajet de rayonnement respectif s'étendant de la LED associée (504) jusqu'à une zone d'irradiation (556), chaque LED de la pluralité de LED (504) étant configurée pour émettre des rayons UV afin de stimuler une production de vitamines D dans la peau d'un sujet humain ;
une pluralité de diffuseurs (520), dans lequel un diffuseur (520) de la pluralité de diffuseurs (520) est situé le long de chacun des trajets de rayonnement en aval des LED respectives (504) ; et
une pluralité de collimateurs (526), dans lequel un collimateur (526) de la pluralité de collimateurs (526) est situé le long de chacun des trajets de rayonnement en aval des diffuseurs respectifs (520).

2. Source de rayonnement (500a) selon la revendication 1, dans lequel la pluralité de LED (504) forme un panneau de sources concentrées qui, chacune, produisent une distribution commune des rayons UV.

3. Source de rayonnement (500a) selon l'une quelconque des revendications précédentes, dans lequel la pluralité des LED (504) est agencée en configuration de réseau.

4. Source de rayonnement (500a) selon l'une quelconque des revendications précédentes, dans lequel les LED de la pluralité des LED (504) sont espacées les unes des autres de sorte que le rayonnement émanant d'une LED (504) chevauche le rayonnement émis par une LED adjacente (504).

5. Source de rayonnement (500a) selon l'une quelconque des revendications précédentes, dans lequel le boîtier est configuré pour intégrer la pluralité de LED (504) selon un agencement vertical, la zone d'irradiation (556) étant configurée pour recevoir au moins une partie du corps du sujet humain, dans lequel la partie du corps se présente selon un agencement vertical correspondant.

6. Source de rayonnement (500a) selon l'une quelconque des revendications précédentes, dans lequel le boîtier définit la zone d'irradiation, dans lequel la zone d'irradiation est configurée pour recevoir une partie du sujet humain.

7. Source de rayonnement (500a) selon l'une quelconque des revendications précédentes, dans lequel :
la pluralité des LED (504) est configurée pour diriger, au moins en partie, des rayons UV collimatés le long du trajet de rayonnement ;
la pluralité de diffuseurs (520) réduit la collimation des rayons UV émanant de la pluralité des LED (504) ; et
la pluralité des collimateurs (526) augmente la collimations des rayons UV émanant de la pluralité des LED (504).

8. Source de rayonnement (500a) selon l'une quelconque des revendications précédentes, dans lequel chaque trajet de rayonnement comporte un réflecteur situé en aval de la LED respective (504), le réflecteur étant configuré pour diriger les rayons UV le long du trajet de rayonnement respectif.

9. Source de rayonnement (500a) selon l'une quelconque des revendications précédentes, comprenant en outre un filtre (518) situé le long de chaque trajet de rayonnement lequel est configuré pour, de préférence, permettre le passage de rayons UV à une longueur d'onde de 297 nm environ par rapport à des rayons UV à des longueurs d'onde supérieures à 304 nm ou inférieures à 292 nm.

10. Source de rayonnement (500a) selon la revendication 9, dans lequel ledit filtre (518) intègre un film.

11. Source de rayonnement (500a) selon l'une quelconque des revendications précédentes, comprenant en outre une pluralité de réflecteurs configurés pour réfléchir les rayons UV générés à hauteur de la pluralité des LED, le rayonnement réfléchi étant dirigé le long du trajet de rayonnement respectif s'étendant de la LED respective vers la zone d'irradiation.

12. Source de rayonnement (500a) selon les revendications 1 à 10, comprenant en outre un filtre situé le long de chaque trajet de rayonnement lequel est positionné en aval de la LED respective.

13. Source de rayonnement (500a) selon l'une quelconque des revendications 1 à 11, comprenant en outre un filtre situé le long de chaque trajet de rayonnement lequel est positionné en aval du collimateur respectif.

14. Source de rayonnement (500a) selon l'une quelconque des revendications précédentes, comprenant en outre une pluralité de lentilles (506) fixée sur les bandes à émission latérale respectives de la pluralité de LED (504).

15. Source de rayonnement (500a) selon l'une quelconque des revendications précédentes, comprenant en outre une lentille située en aval de chaque LED de la pluralité de LED (504).
